Europäisches Patentamt
European Patent Office
Office européen des brevets

(19) (11) Publication number: **0 138 662 A2**

(12) **EUROPEAN PATENT APPLICATION**

(21) Application number: **84401808.5**

(22) Date of filing: **12.09.84**

(51) Int. Cl.⁴: **C 07 D 237/24**, A 01 N 43/58

(30) Priority: **14.09.83 US 532019**

(43) Date of publication of application: **24.04.85 Bulletin 85/17**

(84) Designated Contracting States: **AT BE CH DE FR GB IT LI LU NL SE**

(71) Applicant: **LAFARGE COPPEE, 28, rue Emile Ménier, F-75782 Paris Cedex 16 (FR)**

(72) Inventor: **Labovitz, Jeffrey Niles, 145 Waverley Street, Palo Alto California 94301 (US)**
Inventor: **Fang, Lawrence, 225 Coronado Ave No. 220, Daly City California (CA) 94015 (US)**

(74) Representative: **Phélip, Bruno et al, c/o Cabinet Harlé & Phélip 21, rue de la Rochefoucauld, F-75009 Paris (FR)**

(54) **Pollen suppressant comprising a 3-carboxyalkoxyaminocarbonylpyridazine.**

(57) Pollen suppressants of the formula

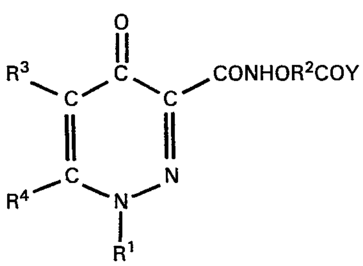

wherein $R^1$ and $R^4$ independently represent $C_1$-$C_4$ alkyl, phenyl, naphthyl, or phhenyl or naphthyl substituted with one to three substituents selected from the group consisting of halogen, trihalomethyl, $C_1$-$C_4$ alkoxy, $C_1$-$C_4$ alkyl, and cyano; $R^2$ is a divalent alkyl, alkenyl, or alkynyl radical having 0 to 4 multiple bonds and containing 1 to 21 carbon atoms if linear and 1 to 5 linear carbons substituted with 1 to 3 alkyl substituents of 1 to 4 carbon atoms if branched or a divalent cycloalkyl; cycloalkenyl containing 1 or 2 double bonds; phenyl; or alkyl- or dialkylcycloalkyl, -cycloalkenyl, or -phenyl radical containing 3 to 10 carbon atoms and having a ring containing 3 to 7 carbon atoms; Y is OH, $OY^1$ wherein $Y^1$ is an agronomically acceptable metal ion, $OR^5$ wherein $R^5$ is a $C_1$-$C_4$ alkyl radical, or $NR^6R^7$ wherein $R^6$ and $R^7$ independently represent hydrogen or $R^5$ or $R^6$ and $R^7$ taken together represent a $C_4$-$C_5$ divalent alkyl or alkenyl group which forms a ring when taken together with the nitrogen of $NR^6R^7$; and $R^3$ is hydrogen, a halogen, a $C_1$-$C_4$ alkyl group, a carboxy group or an agronomically acceptable alkali metal salt thereof, or a group of the formula $-COOR^5$ or $-CONR^5R^6$ where $R^5$ is a $C_1$-$C_4$ alkyl group and $R^6$ is $R^5$ or hydrogen, are disclosed along with methods of producing these compounds and of using them to produce hybrid seeds in self-fertilizing plants.

EP 0 138 662 A2

1286-003-30
38/

TITLE OF THE INVENTION

POLLEN SUPPRESSANT COMPRISING A
3-CARBOXYALKOXYAMINOCARBONYLPYRIDAZINE

BACKGROUND OF THE INVENTION

Field of the Invention:

The present invention relates to a series of pyridazinone compounds, a process for their preparation, compositions containing these compounds, and a method of regulating the growth of plants using such compounds.

Description of the Prior Art:

Although genetic manipulation of plants through cross-breeding is a well-known process, hybrids of self-pollinating plants had been difficult to produce. In some cases, *e.g.*, corn, intensive hand labor is required to prevent self-pollinating but is possible because the male and female flower parts are distant from each other on the corn stalk. However, in other plants, *e.g.*, wheat, the male and female plant parts are contained within the same flower and self-pollination is difficult if not impossible to prevent. In wheat, the male stamen produces pollen inside a closed flower. The pollen then falls within the closed flower onto the female stigma. Only after

this self-pollination step does the flower open to release extra pollen. Mechanical prevention of self-pollination as is practiced in corn is accordingly impossible in a plant such as wheat.

Nevertheless, it is possible to inhibit self-pollination in wheat and similar plants by chemically inhibiting the formation of pollen or by inducing the plant to produce non-functioning pollen. Several compounds have previously been developed which produce these effects.

DOS 28 08 795 discloses compounds of the formula:

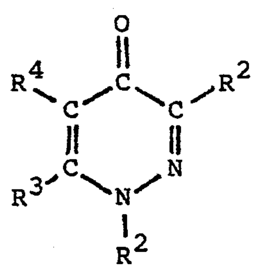

in which $R^1$ is carboxy, a carboxy salt, or an alkoxy carbonyl group, $R^2$ is a substituted phenyl group, $R^3$ is alkyl, and $R^4$ is hydrogen, alkyl or halogen. These compounds are disclosed to be pollen suppressants.

Published European Patent Application 0 037 133 discloses compounds of the formula:

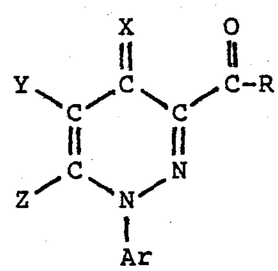

in which X represents oxygen or sulfur, Y represents hydrogen, halogen or an alkyl group, Z represents an alkyl group, Ar represents an optionally substituted phenyl group, and R represents a group which may be, among others $NR^1R^2$ or $ONR^1R^2$ in which $R^1$ can be hydrogen and $R^2$ can be an alkoxy group, an acyl group derived from a carboxylic or carbamic acid, or an alkyl group substituted with a carboxylic acid or ester group. These compounds are also disclosed to be pollen suppressants.

Published European Patent Application 0 049 971 discloses compounds of the formula:

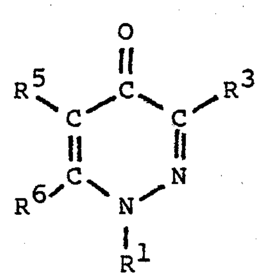

in which $R^1$ can be phenyl substituted with a halogen, $R^3$ can be carboxy or an alkali metal salt thereof, an alkoxy carbonyl, or a substituted carbamoyl, $R^5$ is a carboxy derivative of the type defined for $R^3$, and $R^6$ is a $C_1$-$C_4$ alkyl group. These compounds are disclosed to be chemical hybridizing agents which operate by causing male plant sterility.

Nevertheless, many of these compounds have adverse affects on hybrid seed quality or injure plants at doses only slightly above those required to produce maximum male plant sterility. Accordingly, a continued need for new pollen suppressants useful for producing hybrid seed of cereal grain exists.

## SUMMARY OF THE INVENTION

Accordingly, it is an object of this invention to provide chemical sterilants for producing hybrid seed of cereal grain plants.

It is a further object of this invention to provide a method of suppressing pollen production in cereal grain plants using these compounds.

It is still a further object of this invention to provide a method for producing hybrid seed of cereal grain plants using the novel chemical sterilants of the invention.

These and other objects of the invention as will hereinafter become more readily apparent have been accomplished by providing a chemical pollen suppressant of the formula:

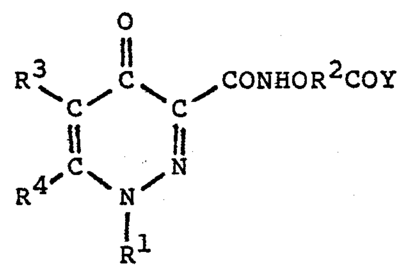

wherein $R^1$ and $R^4$ independently represent $C_1$-$C_4$ alkyl, phenyl, naphthyl, or phenyl or naphthyl substituted with one to three substituents selected from the group consisting of halogen, trihalomethyl, $C_1$-$C_4$ alkoxy, $C_1$-$C_4$ alkyl, and cyano;

$R^2$ is a divalent alkyl, alkenyl, or alkynyl radical having 0 to 4 multiple bonds and containing 1 to 21 carbon atoms if linear and 1 to 5 linear carbons substituted with 1 to 3 alkyl substituents of 1 to 4 carbon atoms if branched or a divalent cycloalkyl; cycloalkenyl containing 1 or 2 double bonds; phenyl; or alkyl- or dialkylcycloalkyl, -cycloalkenyl, or -phenyl radical containing 3 to 10 carbon atoms and having a ring containing 3 to 7 carbon atoms;

Y is OH, $OY^1$ wherein $Y^1$ is an agronomically acceptable metal ion, $OR^5$ wherein $R^5$ is a $C_1$-$C_4$ alkyl

radical, or $NR^6R^7$ wherein $R^6$ and $R^7$ independently represent hydrogen or $R^5$ or $R^6$ and $R^7$ taken together represent a $C_4$-$C_5$ divalent alkyl or alkenyl group which forms a ring when taken together with the nitrogen of $NR^6R^7$; and

$R^3$ is hydrogen, a halogen, a $C_1$-$C_4$ alkyl group, a carboxy group or an agronomically acceptable alkali metal salt thereof, or a group of the formula $-COOR^5$ or $-CONR^5R^6$ where $R^5$ is a $C_1$-$C_4$ alkyl group and $R^6$ is $R^5$ or hydrogen.

## DESCRIPTION OF THE PREFERRED EMBODIMENTS

The present invention provides novel pyridazoylamines in which the substituent at the 3-position of the pyridazinone ring is not a substituent normally used in pollen supressants. Instead, $R^2$ is a carboxyalkoxyaminocarbonyl group. Thus, the chemical pollen suppressants of the invention include those compounds having the formula:

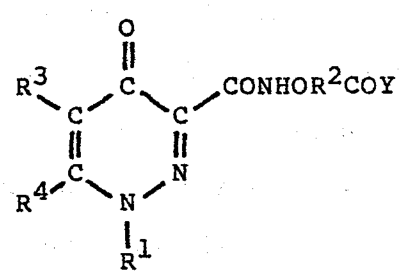

wherein $R^1$ and $R^4$ independently represent $C_1$-$C_4$ alkyl, phenyl, naphthyl, or phenyl or naphthyl substituted with one to three substituents selected from the group consisting of halogen, trihalomethyl, $C_1$-$C_4$ alkoxy, $C_1$-$C_4$ alkyl, and cyano;

$R^2$ is a divalent alkyl, alkenyl, or alkynyl radical having 0 to 4 multiple bonds and containing 1 to 21 carbon atoms if linear and 1 to 5 linear carbons substituted with 1 to 3 alkyl substituents of 1 to 4 carbon atoms if branched or a divalent cycloalkyl; cycloalkenyl containing 1 or 2 double bonds; phenyl; or alkyl- or dialkylcycloalkyl, -cycloalkenyl, or -phenyl radical containing 3 to 10 carbon atoms and having a ring containing 3 to 7 carbon atoms;

Y is OH, $OY^1$ wherein $Y^1$ is an agronomically acceptable metal ion, $OR^5$ wherein $R^5$ is a $C_1$-$C_4$ alkyl radical, or $NR^6R^7$ wherein $R^6$ and $R^7$ independently represent hydrogen or $R^5$ or $R^6$ and $R^7$ taken together represent a $C_4$-$C_5$ divalent alkyl or alkenyl group which forms a ring when taken together with the nitrogen of $NR^6R^7$; and

$R^3$ is hydrogen, a halogen, a $C_1$-$C_4$ alkyl group, a carboxy group or an agronomically acceptable alkali metal salt thereof, or a group of the formula $-COOR^5$ or $-CONR^5R^6$ where $R^5$ is a $C_1$-$C_4$ alkyl group and $R^6$ is $R^5$ or hydrogen.

Preferred substituents are those in which $R^1$ represents phenyl or phenyl substituted with one to three substituents selected from the group consisting of halogen, trihalomethyl, $C_1$-$C_4$ alkoxy, $C_1$-$C_4$ alkyl and cyano; more preferably phenyl substituted with one of said substituents; even more preferably phenyl substituted with one halogen atom; and most preferably phenyl substituted with chlorine in the para position; $-R^2CO-$ is $-CH_2(CH_2CH_2)_nCO-$, where n is an integer from 0 to 2, or the residue of a naturally occurring fatty acid, more preferably is $-CH_2(CH_2CH_2)_nCO-$, and most preferably is $-CH_2CO-$; Y is OH or $OY^1$, where $Y^1$ is preferably an alkali metal ion and most preferably is sodium or potassium, and most preferably Y is $OY^1$ where $Y^1$ is sodium or potassium; $R^3$ is hydrogen; and $R^4$ is a $C_1$-$C_4$ alkyl group, most preferably a methyl group.

Preferred compounds are defined by selecting one or more of these listings of preferred substituents in combination with the general formula previously given. Certain combinations of substituents are especially preferred. One preferred grouping occurs when $R^1$ is phenyl mono-substituted with a halogen, $R^2$ is $-CH_2-$, $R^3$ is hydrogen and $R^4$ is methyl.

Also included within the scope of the invention are agronomically acceptable acid addition salts of compounds having the general formula given. Typical

acid addition salts are those formed with strong acids such as hydrochloric, hydrobromic, sulfuric, and nitric acids. Salts of acidic or basic functional groups in the substituents are also included in this invention. By agronomically acceptable salt, here and elsewhere in this application, is meant that the salt is not significantly more toxic to the plant or to a consumer of the plant to which the salt is applied than the compound from which the salt is formed.

Typical compounds of the invention include the following:

1-phenyl-1,4-dihydro-3-carboxymethyloxyaminocarbonyl-4-oxo-6-methylpyridazine

1-phenyl-1,4-dihydro-3-[3-(dimethylaminocarbonyl)propyloxyaminocarbonyl]-4-oxo-6-ethylpyridazine

1-phenyl-1,4-dihydro-3-(17-carboxyheptadecyloxyaminocarbonyl)-4-oxo-6-phenylpyridazine

1-(4-chlorophenyl)-1,4-dihydro-3-pyrrolidinamidomethyloxyaminocarbonyl-4-oxo-6-methylpyridazine

1-(4-bromophenyl)-1,4-dihydro-3-(4-carboxylphenylmethyloxyaminocarbonyl)-4-oxo-6-ethylpyridazine-5-carboxylic acid

1-(3,4-dichlorophenyl)-1,4-dihydro-3-(17-carboxy-9-pentadecenyloxyaminocarbonyl)-4-oxo-6-propylpyridazine

1-(4-iodophenyl)-1,4-dihydro-3-(3-ethyloxycarbonyl-2-methylpropyloxyaminocarbonyl)-4-oxo-6-butylpyridazine

1-(4-fluorophenyl)-1,4-dihydro-3-(2-carboxycyclopropyloxyaminocarbonyl)-4-oxo-6-butylpyridazine

1-(4-chlorophenyl)-1,4-dihydro-3-carboxymethyloxyaminocarbonyl-4-oxo-6-phenylpyridazine

1-(3-chlorophenyl)-1,4-dihydro-3-[4-(carbamoylmethyl)-2,5-cyclohexadienyloxyaminocarbonyl]-4-oxo-6-methylpyridazine

1-(2,4,6-trichlorophenyl)-1,4-dihydro-3-(3-carboxymethylcyclopentylmethyloxyaminocarbonyl)-4-oxo-6-phenylpyridazine

1-(4-methylphenyl)-1,4-dihydro-3-(3-carboxypropyloxyaminocarbonyl)-4-oxo-6-phenylpyridazine

1,(4-trifluoromethylphenyl)-1,4-dihydro-3-(5-carboxypentyloxyaminocarbonyl)-4-oxo-6-methyl-pyridazine

1-(3-ethoxyphenyl)-1,4-dihydro-3-(17-carboxy-3,6,9-heptadecatrienyloxyaminocarbonyl)-4-oxo-6-ethyl-pyridazine

1-(3-cyanophenyl)-1,4-dihydro-3-methyloxycarbonylmethyloxyaminocarbonyl-4-oxo-6-butylpyridazine

1-(2-chloro-4-methylphenyl)-1,4-dihydro-3-carbamoylmethyloxyaminocarbonyl-4-oxo-6-phenylpyridazine

1-(2-trifluoromethyl-4-chlorophenyl)-1,4-dihydro-3-carboxymethyloxyaminocarbonyl-4-oxo-6-methylpyridazine

1-(2-trifluoromethyl-4-bromophenyl)-1,4-dihydro-3-carboxymethyloxyaminocarbonyl-4-oxo-6-ethylpyridazine-3-carboxylic acid

1-(2-chloro-5-trifluoromethylphenyl)-1,4-dihydro-3-pyrrolidinamidomethyloxyaminocarbonyl-4-oxo-6-ethyl-pyridazine-5-carboxylic acid

1-(2-naphthyl)-1,4-dihydro-3-piperidinamidomethyloxyaminocarbonyl-4-oxo-6-butylpyridazine-5-carboxylic acid

and the sodium, potassium, and lithium carboxylate salts of each of the above compounds and the acid addition salts of each of the above listed compounds. By carboxylate salt is meant a salt of a carboxylate group in a substituent at C-3 or C-5. By acid addition salt is meant a salt formed by the protonation of a ring nitrogen.

The compounds in the invention can be synthesized according to known methods for the production of analogous compounds or can be produced by synthetic modification of known pyridazinones. For examples, one suitable method involves the reaction of a 4-hydroxy-2-pyrone of the formula:

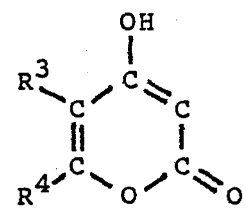

in which $R^3$ and $R^4$ represents one of the groups previously named, with a diazonium salt, for example, a diazonium chloride, prepared from an amine of the formula $R^1NH_2$ where $R^1$ has the meaning previously defined. The reaction is carried out by reacting the pyrone with one equivalent of an aqueous base, such as potassium or sodium hydroxide, acetate, or carbonate, generally at a temperature of from -10 - 50°C in a polar solvent, such as water, methanol, ethanol, or dimethyl formamide. A product having the following formula is obtained:

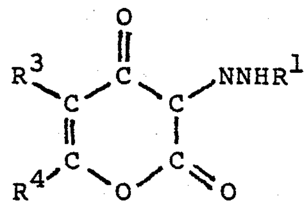

wherein $R^1$, $R^3$ and $R^4$ have the previously given meanings. Upon heating at a temperature of from 20 to 150°C (preferably 40 to 100°C) in an aqueous solution of acid or base, such as hydrochloric acid, trifluoroacetic acid, sulfuric acid, methanesulfonic acid, nitric acid, sodium carbonate, or sodium hydroxide, a pyridazinone of the formula:

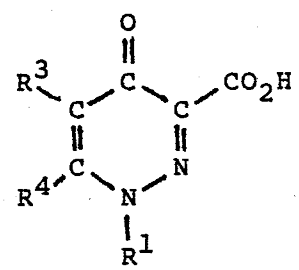

is obtained, where $R^1$, $R^3$, and $R^4$ have the meanings previously defined.

Another suitable synthetic technique is described by Plescia et al, J. Heterocyclic Chem., 18, 333-334 (1981), which is herein incorporated by reference. This method, which involves reaction of azo derivatives of β-dicarbonyl compounds with dimethylformamide dimethylacetal to yield 3-carboxypyridazinones, gives

compounds of the desired formula and can be modified to provide a variety of substituents by relection of the starting materials.

The above-indicated 3-carboxypyridazinone can then be converted into compounds of the invention by known methods. For example, the carboxylic acid group can be converted into a mixed acid anhydride, for example by reacting with ethylchloroformate. The acid anhydride is then converted into an amide by reacting the acid anhydride with a group of the formula $NH_2OR^2CO_2R^5$. This series of reactions is summarized in the following scheme:

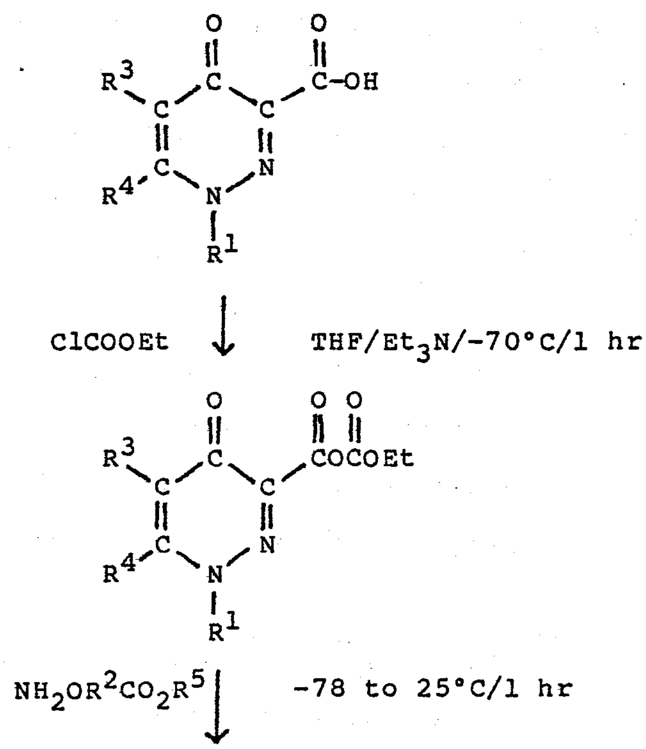

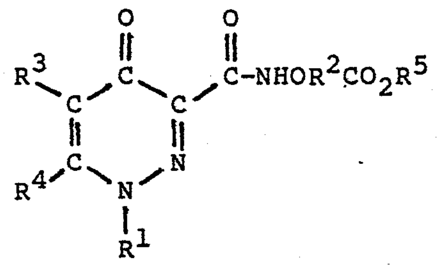

The reactant of formula $NH_2OR^2CO_2R^5$ can be synthesized by conventional techniques. For example, an alkyl halide of the formula $XR^2CO_2R^5$ where X is a halogen, preferably Cl or Br, is reacted with N-hydroxyphthalimide to produce an O-alkylated N-hydroxyphthalimide which is then hydrolyzed in aqueous base to release $NH_2OR^2CO_2R^5$ or the equivalent carboxylate salt. Conversions of the carboxyl or carboxylate group into other types of acid derivatives, such as amides, are well-known reactions. If desired, the carboxylate group may be present in protected form during the initial stages of reaction, for example as a nitrile, $XR^2CN$. Conventional variations of this technique, as well as many other straightforward synthetic techniques, can be used to produce all variations of the $-CONHOR^2COY$ side chain. For example, many side chain precursors are known compounds (e.g., aminooxyaromatic carboxylates), and the techniques available for synthesizing these known compounds can be easily varied by one skilled in the art of organic

synthesis to produce any reactant of the formula $NH_2OR^2CO_2R^5$.

Various modifications of these reactions can be used to produce all the compounds of the present invention, for example as is disclosed in the three prior art patents previously cited (DOS 28 08 795, EP 37 133, and EP 49 971), which are herein incorporated by reference.

Compounds of the invention are useful as chemical hybridization agents in gramineous crops, such as wheat, barley, maze, rice, sorgrum, millet, oats, rye, triticale, forage crops and the like. Treatment of wheat is particularly preferred. Different plant growth regulating effects will be obtained depending upon the growth stage of the plant when treated. Compounds of the invention induce selected male sterility without also inducing unacceptable female sterility. About 30% female fertility is generally acceptable, although this level may differ when the method is used commercially, based on the economics of $F_1$ seed production. As used herein, the term male sterility includes sterility caused by lack of male flower parts, by formation of sterile pollen, and by male flower parts which produce normal pollen but are functionally unable to cause pollination. Where the male sterility of compounds of the invention is

accompanied by female infertility of an unacceptable level or by phytotoxicity, the compounds are still minimally useful in production of ergot, for example as described in French Published Patent Application No. 2400832, which is herein incorporated by reference.

When compounds of the invention are used in hybridization, they are used in an amount sufficient to produce the effect of male sterility without producing a phytotoxic reaction or other undesired side-reaction. Compounds of the invention are generally applied at a rate of from 0.025 to 20.0 pounds per acre, and preferably from 0.125 to 10.0 pounds per acre. The amount used depends upon the plant type and the method of application as is well-known to those skilled in the art and can be determined by simple experimentation if not known.

Although any method of hybridization may be used, the following method generally is sufficient. The two parent strains to be crossed are planted in alternate sections, rows, or groups of rows. The female parent is treated with a compound of the invention in order to render this parent male sterile. Pollen from the male (untreated) parent then fertilizes the female parent, either by means of human intervention or preferably by means of a natural process, such as wind-borne pollination. The seed produced by the female parent is

an F-1 hybrid, which is then collected according to convention techniques.

One method of applying the compounds of the invention in the previously-mentioned hybridization technique or for otherwise inducing male sterility is application directly to the plant leaves. When this method is used, very selective male sterility can be obtained when the compound is applied between the beginning of bloom and the beginning of meiosis.

Compounds of the invention can also be applied directly to seed in order to cause male sterility, whereby the seeds are dipped into a fluid formulation containing the active ingredient. Seed can also be sprayed with a solution or suspension containing a compound of the invention. In general, seed are treated with a compound of the invention in an amount of from about 1/4 to 10 pounds per 100 pounds of seed. Compounds of the invention are also effective when they are applied to the medium in which plants are grown such as soil or the water surface in a rice field.

Compounds of the invention can be used as hybridization materials together with other plant regulatory agents, for example, in mixtures with these compounds. Examples of plant regulating materials which can be used include auxins, gibberellins, ethylene

liberating materials such as Ethephon, pyridones, cytokinins, maleic hydrazide, carbonic acid, 2,2-dimethyl hydrazide, cholines (as well as their salts), (2-chloroethyl)trimethylammonium chloride, triiodobenzoic acid, tributyl-2,4-dichlorobenzene-phosphonium chloride, polymeric N-vinyl-2-oxazolidinones, tri(dimethylaminoethyl)phosphate, and salts of these compounds as well as N-dimethylamino-1,2,3,6-tetrahydrophthalamides and their salts. Compositions containing one or more compounds of the invention in a 1:99-99:1 ratio to one or more different compounds having plant regulatory activities may be prepared. Likewise, compounds of the invention may be prepared into compositions useful for other agricultural purposes, such as herbicides, fungicides, insecticides, and plant bactericides.

A compound of the invention can be applied to a plant either as itself or in combination with other plant growth regulators. A composition containing a compound of the invention and any other active ingredient may be diluted with an agronomically suitable carrier, which is any substance which itself is without any significant effect on plants but which is added in order to allow simpler application of the active ingredients to plants. Carriers include both liquids and solids. Accordingly, compositions of the

invention can be either solid or liquid formulations or solutions. For example, the compounds can be used in powders, emulsifiable concentrates, dusts, pellets, aerosols and solutions. In any of the various formulations, a surface active agent may be added in order to increase uptake of the active compounds. It is especially preferred, and particular for methods which involve application to leaves, to utilize agents which aid in the application of the material, for example, dispersion agents and detergents.

Compounds of the invention can be dissolved in any suitable solvent. Examples of solvents which can be used include water, alcohols, ketones, aromatic hydrocarbons, halogenated hydrocarbons, dimethylformamide, dioxane, and dimethylsulfuloxide. Mixtures of these solvents can likewise be used. The concentration of these solutions can be from about 2 to about 98% by weight of active ingredient and is preferred to be in the range from about 20 to about 75% by weight.

In order to produce emulsifiable concentrates, the compounds of the invention are dissolved in an organic solvent, such as benzene, toluene, xylene, methylated naphthalene, corn oil, terpentine, o-dichlorobenzene, isophorone, cyclohexane, or methyl oleate or in mixtures of these solvents, together with an

emulsifying material which allows the dispersion in water. Suitable emulsifying agents include ethylene oxide derivatives of alkylphenols or long-chained alcohols, mercaptans, carboxylic acids, and reactive amines, and especially high molecular weight alcohols. Solvent-soluble sulfates or sulfonates, such as the alkaline earth salts or amine salts of alkylbenzenesulfonates as well as sodium fatty alcohol sulfates with surface active properties can be utilized as emulsifying agents either alone or in combination with an ethylene oxide reaction product. Free-flowing emulsion concentrates are formulated similarly to emulsifiable concentrates and contain, in addition to the previously described components, water as well as a stabilizing agent, such as a water-soluble cellulose derivative or a water-soluble salt of a polyacrylic acid. The concentration of the active ingredient in the emulsifiable concentrate is generally about 10 to 60 wt. % and in free-flowing emulsion concentrates is generally about 10 to 60% or sometimes up to 75% by weight.

When a powder containing the compound of the invention is being prepared, the active ingredient is usually mixed with a finely divided solid, such as a clay, an organic silicate or carbonate, or a silica gel along with an agent capable of holding together the

resulting materials. The concentration of the active ingredient in such powders generally lies between about 20 and 98% by weight and preferably lies between 40 and 75% by weight. A dispersion material can generally be present in an amount of about 0.5 to 3% by weight of the entire powder. An agent may be added in order to control water absorption and if added is generally present in an amount of about 0.1 to about 5% by weight of the total powder.

Dusts can be prepared by mixing the active ingredient with a finely divided inert solid, which can be of an organic or inorganic nature. Suitable material for this purpose include flour, farina, diatomite, silicates, carbonates, and clays. A satisfactory method for the production of dusts involves crushing a wettable powder together with a finely divided carrier. A dust concentrate, which contains from about 20 to about 80% of the active ingredient, is produced according to known methods and then diluted to form a final concentration of the compound of the invention of about 1 to about 10% by weight of the dust.

Particulate formulations can be prepared by any known method, for example by impregnating the active ingredient into a solid material, such as particulate Fullers earth, vermiculite, cornmeal, seed hulls such

as grain hulls, or other materials. A solution of one or more of the compounds of the invention in a freely flowing organic solvent can be applied to the particulate solid or mixed therewith, after which the solvent is evaporated away. The particulate material is not limited to a particular size. However, a useful size is from 16 to 60 mesh (U.S. standard mesh size). The active ingredient generally occupies about 2 to about 15 wt % of the particulate formulation.

Salts of the compounds of the invention can be prepared as aqueous solutions and applied in this form. The salts occupy typically about 0.05 to about 50 wt. % and preferably from about 0.1 to 10 wt. % of the solution. In any event, these solutions may be diluted with additional water prior to use. In some cases the activity of the active material can be increased by including another agent in the solution, such as glycerin, methylethylcellulose, hydroxyethyl cellulose, polyoxyethylene sorbital mono-oleate, polypropylene glycol, polyacrylic acid, polyethylene sodium malonate, or polyethyleneoxide. The auxiliary occupies generally from about 0.1 to about 5 wt. % and particularly from about 0.5 to 2 wt. % of the solution. The various solutions can in any case also contain an agriculturally suitable surface active agent.

The compounds of the invention can be applied according to any known methods, for example in the form of hydraulic sprays, air sprays or dusts. For methods which involve the application of small volumes, a solution of the compound is generally utilized. The volume used and the rate of application depend upon various factors which vary with the method used, such as the specific type of application method, the stage of development of the plant to which the active ingredient is being applied, and other factors well known to those skilled in the art or easily determined by simple experimentation.

Having now generally described this invention, the same will be better understood by reference to certain specific examples which are included herein for purposes of illustration only and are not intended to be limiting of the invention or any embodiment thereof, unless so specified.

Example 1: Synthesis of 1-(4-Chlorophenyl)-1,4-dihydro-3-carboxymethyloxyaminocarbonyl-4-oxo-6-methylpyridazine

To a mixture of 2.6 g (10 mmole) of 1-(p-chlorophenyl)-1,4-dihydro-4-oxo-6-methylpyridazine-3-carboxylic acid and 1.4 ml of triethylamine in 40 ml of dry THF was added at -40°C 1.06 ml (11 mmole) of ethyl chloroformate. The reaction mixture was stirred while

the temperature was allowed to reach 20°C over 30 minutes. The temperature was then lowered to -20°C, and there was added a mixture of 1.35 g (11 mmole) of methyl aminoxyacetate hydrochloride and 2.1 ml of triethylamine in 20 ml of THF. The reaction was then stirred for 2 hours at room temperature, diluted with 100 ml of ether and washed in sequence with 1N hydrochloric acid, saturated sodium bicarbonate and brine. After drying and evaporation of solvent there was obtained 1.8 g of crude product which was saponified without purification using 0.3 g of potassium hydroxide in 15 ml of methanol. The crude potassium salt obtained on removal of solvent was treated with 1N hydrochloric acid until a pH of 3-4 was reached. The solid acid thus obtained was collected and dried (1.22 g, 36%) m.p. 208-210°.

Example 2: Biological Activity

A biological assay for pollen suppression was conducted on the wheat variety W-41 (Anza). This is a heavy tillering wheat which is grown commercially in California. Seeds were planted to provide four plants per 8-inch pot. Plants were raised in a greenhouse until the stage indicated in the following table of results. Three different stages of growth were defined for the purposes of this invention as follows: Stage

1, spike length of 0.1-0.5 cm; Stage 2, spike length of 0.5-1.5 cm; Stage 3, spike length of 1.5-2.5 cm. External appearance was correlated with the development of the spikelet in order to avoid mistaking the onset of meiosis. Spikelets were removed at various developmental stages and anthers were removed from the most mature florets (which generally occured in about the middle of the spiklet). The anthers were crushed in acetocarmine or propeocarmine and the state of pollen development was assessed. Cytological examinations were made to assess the best time for application. Compounds were applied as solutions in water or water/acetone (5-50% acetone) or as aqueous emulsions. In all cases, 0.1% Triton X-100 was used as a wetting agent. Plants were sprayed to runoff with a test solution and then replaced in such a way that control plants were interspersed with treated plants. Heads were bagged upon emergence and seed set was used as a measure of sterility induction. Compounds that demonstrated good sterilization ability were tested for their effect on female ferility by cross-pollination of awned female plants with awnless male pollen donors.

Control studies were conducted using a known prior art compound (1-(4-chlorophenyl)-1,4-dihydro-4-oxo-6-methylpyridazine-3-carboxylic acid). Optimal dosage and correct stage of application of this compound were

determined in order that test crosses could be compared to test crosses made using the compounds of the invention.

Using the general procedure described above, 1-(4-chlorophenyl)-1,4-dihydro-3-methylsulfonamidocarbonyl-4-oxo-6-methylpyridazine was screened for pollen suppressing activity.

Results of Bioassay

| Stage of Application | Dose (ppm) | % Sterility |
|---|---|---|
| 2 | 500 | 100 |
| 2 | 250 | 100 |
| 2 | 125 | 100 |
| 2 | 62.5 | 51.6 |
| 2 | 31.25 | 19.8 |

The invention now being fully described, it will be apparent to one of ordinary skill in the art that many changes and modifications can be made thereto without departing from the spirit or scope of the invention as set forth herein.

CLAIMS

1. A pollen suppressant of the formula

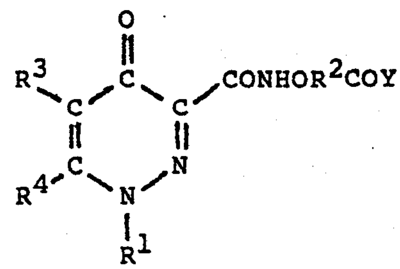

wherein $R^1$ and $R^4$ independently represent $C_1$-$C_4$ alkyl, phenyl, naphthyl, or phenyl or naphthyl substituted with one to three substituents selected from the group consisting of halogen, trihalomethyl, $C_1$-$C_4$ alkoxy, $C_1$-$C_4$ alkyl, and cyano;

$R^2$ is a divalent alkyl, alkenyl, or alkynyl radical having 0 to 4 multiple bonds and containing 1 to 21 carbon atoms if linear and 1 to 5 linear carbons substituted with 1 to 3 alkyl substituents of 1 to 4 carbon atoms if branched or a divalent cycloalkyl; cycloalkenyl containing 1 or 2 double bonds; phenyl; or alkyl- or dialkylcycloalkyl, -cycloalkenyl, or -phenyl radical containing 3 to 10 carbon atoms and having a ring containing 3 to 7 carbon atoms;

Y is OH, $OY^1$ wherein $Y^1$ is an agronomically acceptable metal ion, $OR^5$ wherein $R^5$ is a $C_1$-$C_4$ alkyl radical, or $NR^6R^7$ wherein $R^6$ and $R^7$ independently

represent hydrogen or $R^5$ or $R^6$ and $R^7$ taken together represent a $C_4$-$C_5$ divalent alkyl or alkenyl group which forms a ring when taken together with the nitrogen of $NR^6R^7$; and

$R^3$ is hydrogen, a halogen, a $C_1$-$C_4$ alkyl group, a carboxy group or an agronomically acceptable alkali metal salt thereof, or a group of the formula $-COOR^5$ or $-CONR^5R^6$ where $R^5$ is a $C_1$-$C_4$ alkyl group and $R^6$ is $R^5$ or hydrogen.

2. The pollen suppressant of Claim 1, wherein $R^1$ is phenyl or phenyl substituted with 1 to 3 substituents selected from the group consisting of halogen, trihalomethyl, $C_1$-$C_4$ alkoxy, $C_1$-$C_4$ alkyl, and cyano.

3. The pollen suppressant of Claim 2, wherein $R_1$ is phenyl substituted with one substituent selected from the group consisting of halogen and trihalomethyl.

4. The pollen suppressant of Claim 3, wherein said substituent is halogen.

5. The pollen suppressant of Claim 4, wherein said halogen is chlorine.

6. The pollen suppressant of Claim 5, wherein $R^1$ is 4-chlorophenyl.

7. The pollen suppressant of Claim 1, wherein $R^3$ is hydrogen or a carboxy group or an agronomically acceptable alkali metal salt thereof.

8. The pollen suppressant of Claim 7, wherein $R^3$ is hydrogen.

9. The pollen suppressant of Claim 1, wherein $R^4$ is a $C_1$-$C_4$ alkyl or phenyl group.

10. The pollen suppressant of Claim 9, wherein $R^4$ is a $C_1$-$C_4$ alkyl group.

11. The pollen suppressant of Claim 10, wherein $R^4$ is methyl.

12. The pollen suppressant of Claim 1, wherein $R^1$ is a phenyl substituted with one substituent selected from a group consisting of halogen and trihalomethyl, $R^3$ is hydrogen, and $R^5$ is a $C_1$-$C_4$ alkyl group.

13. The pollen suppressant of Claim 1, wherein $-R^2CO-$ is $-CH_2(CH_2CH_2)_nCO$, where n is an integer from 0 to 2, or the residue of a naturally occurring fatty acid.

14. The pollen suppressant of Claim 13, wherein $-R^2CO-$ is $-CH_2(CH_2CH_2)_nCO-$.

15. The pollen suppressant of Claim 1, wherein $R^1$ is 4-chlorophenyl, $-R^2COY$ is $-CH_2CO_2H$, $R^3$ is hydrogen, and $R^4$ is methyl.

16. A plant-growth-regulating composition comprising at least one compound of Claim 1 in combination with an agronomically acceptable carrier.

17. A method of regulating the growth of a plant, which comprises treating said plant, a seed from which

said plant is to be grown, or a medium in which said plant is growing or is to be grown with a compound of Claim 1.

18. A method of producing hybrid seeds from a self-pollenizing plant which comprises sterilizing the male anthers of a female parent plant with a compound of Claim 1 and pollenating said female parent with pollen from an untreated male parent, thereby producing said hybrid seed.

19. The method of Claim 18, wherein said hybrid seed is seed from wheat, barley, rye, oats, millet, or corn.

20. The method of Claim 19, wherein said seed is wheat seed.

21. A method of regulating the growth of a plant, which comprises treating said plant, a seed from which said plant is to be grown, or a medium in which said plant is growing or is to be grown with a composition of Claim 16.

22. A method of producing hybrid seeds from a self-pollenizing plant which comprises sterilizing the male anthers of a female parent plant with a composition of Claim 16 and pollenating said female parent with pollen from an untreated male parent, thereby producing said hybrid seed.

23. The method of Claim 22, wherein said hybrid seed is seed from wheat, barley, rye, oats, millet, or corn.

24. The method of Claim 23, wherein said seed is wheat seed.